# EUROPEAN PATENT SPECIFICATION

(11) **EP 2 251 323 B1**
(45) Date of publication and mention of the grant of the patent: **23.04.2014**
(21) Application number: 09160276.3
(22) Date of filing: 14.05.2009
(51) Int. Cl.: C07C 253/34, C07C 255/57

(54) **Method for the purification of entacapone**
Verfahren zur Reinigung von Entacapon
Procédé de purification d'entacapone

(43) Date of publication of application: 17.11.2010
(73) Proprietor: F.I.S.- Fabbrica Italiana Sintetici S.p.A., 36075 Alte di Montecchio Maggiore (VI) (IT)
(72) Inventor: Grandini, Cristiano, 44025 Massa Fiscaglia, Ferrara (IT); Leganza, Alessandro, 36040 Torri di Quartesolo, Vicenza (IT); Galvagni, Marco, 37131 Verona (IT); Peruffo, Massimo, 36075 Montecchio Maggiore, Vicenza (IT)
(74) Representative: Long, Giorgio

(56) References cited:
- WO-A-2005/063693
- WO-A-2006/064296
- WO-A-2007/054950
- WO-A-2007/094007

## Description

### Field of the invention

The present invention relates to a method for the purification of Entacapone in the crude form as obtained by a synthesis process in accordance with claim 1 and appended claims.

### Background art

**Entacapone**, (2E)-2-cyano-3-(3,4-dihydroxy-5-nitrophenyl)-N,N-diethyl-2-propenamide, is an inhibitor of cathecol-O-methyltransferase (COMT) and is used in the treatment of Parkinson's disease.

There are various prior art patents disclosing the synthesis of Entacapone.

US 5,446,194 provides for the Knoevenagel condensation of 3,4-dihydroxy-5-nitrobenzaldehyde with 2-cyano-N,N-diethylacetamide to give Entacapone with a yield of 73%. 3,4-dihydroxy-5-nitrobenzaldehyde is obtained by cleavage of the methyl group in 5-nitrovanillin with HBr.

US 2006/258877 discloses an improved synthesis of Entacapone by Knoevenagel condensation as depicted above, wherein the solvent and the catalyst have been optimized.

EP 589 948 discloses the synthesis of 3,4-dihydroxy-5-nitrobenzaldehyde by cleavage with thiophenol.

DE 2307156, Aust. J. Chem., 1976, 29, 1039-50 and Synth. Comm., 20, 3235-3243 (1990) disclose the preparation of 2-cyano-N,N-diethylacetamide.

Different synthesis routes are also known, as described for example in EP 782 559, WO 2005/063693, WO 2006/064296 and EP 1 978 014.

The active compound is trans-Entacapone, but a certain amount (1-2%) of the cis isomer is always present in the crude product.

Purification methods of crude Entacapone have also been described. US 5,446,194 describes the use of ethanol for crystallizing Entacapone; acetic acid or formic acid are used according to EP 426 468; the following solvents have also been proposed: ethyl acetate or ethyl acetate/petroleum ether 5 : 1 or dichloropropane/diisopropylether 4:1 (J. Mol. Struct. 562 (2001), 129-135), chlorine solvents, alcohols, ACN (WO 2007/077572), methanol or ethyl acetate (WO 2008/023380).

A process for the purification of cis-Entacapone with the use of a mixture of methanol and acetic acid is disclosed in the prior-art document WO 2007/054950.

The object of the present invention is to provide a method of crystallization of crude Entacapone that allows to improve the puriry of the product and to minimize the percentage of cis-Entacapone.

This object is achieved by a purification method as depicted in the annexed claims, whose definitions are integral part of the present description.

### Detailed description of the invention

**Entacapone**, (2E) -2-cyano-3-(3,4-dihydroxy-5-nitrophenyl)-N,N-diethyl-2-propenamide, has the following structural formula:

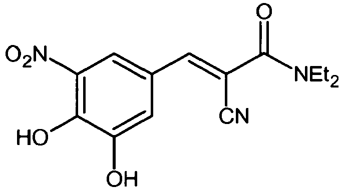

The synthesis of Entacapone can be formed according to one of the synthetic routes known from the prior art. In particular, the process described in U.S. Patent no. 5,446,194 can be used, whose disclosure is herein incorporated by reference. According to this process, Entacapone is obtained by Knoevenagel reaction of 3,4-dihydroxy-5-nitrobenzaldehyde with 2-cyano-N,N-diethylacetamide.

Crude Entacapone isolated therefrom may exemplary have the following purity profile, depending on the batches of product (A%, HPLC):

| | |
|---|---|
| Trans-Entacapone | 96.0-98.0% |
| Cis-Entacapone | 1.0-2.0% |
| Residue of ignition | 0.01-0.05% |

According to the present invention, crude Entacapone has been subjected to a purification step by crystallization. It has been unexpectedly found that by conducting the crystallization in a mixture of solvents composed of toluene and acetone, both yield and purity of the final product can be improved, so that Entacapone with a purity not less than 99.80% and with a content of cis-Entacapone not more than 0.10% or not more than 0.02% (quantitation limit) and any other impurity not more than 0,10% (A%; HPLC validated method) can be obtained.

The mixture of toluene and acetone has a toluene/acetone ratio of between 5:1.5 and 5:6 and the amount of toluene with respect to Entacapone is of between 4.8 and 5.2 volumes of toluene per gram of Entacapone, preferably 5 volumes of toluene per gram of Entacapone.

In one embodiment, the inventive method of purification comprises:
a) dissolving by heating crude Entacapone in a mixture toluene/acetone;
b) distilling off an amount of acetone until an internal temperature of 75-80°C is reached in the Entacapone solution;
c) allowing the Entacapone solution to cool down slowly until Entacapone precipitates;
d) cooling down the Entacapone suspension and isolating pure Entacapone.

Step a) of dissolving by heating crude Entacapone is preferably conducted at reflux temperature.

Step a) may also comprise adding an amount of active carbon, preferably a 5-15% or approximately a 10% amount, in the heated Entacapone solution, keeping under stirring at reflux for a time span, preferably of between 20-40 minutes, and filtering the active carbon off.

In this embodiment, the filter cake is preferably washed with acetone and the resulting acetone phase is added to the Entacapone solution.

In step b), the amount of acetone distilled off is such as to obtain a final toluene/acetone ratio of between 5:1 and 5:3 or of between 5:1.5 and 5:2.

Step d) comprises preferably:
- cooling down the Entacapone suspension to room temperature or to 20°C and keeping under stirring for 30-90 minutes, and subsequently
- cooling down the Entacapone suspension to between -5°C and 5°C or to 0°C and keeping under stirring for 2 to 4 hours.

According to the above purification method, pure Entacapone (trans-Entacapone) can be recovered in a yield of 78-84% and with a purity not less than 99.5% or not less than 99.80%. What is also very important, cis-Entacapone is not more than 0,10% or not more than 0,02% (quantitation limit) and the amount of the major impurity is no more than 0,10% or not more than 0.05% (A%, validated HPLC method).

Accordingly, a further object of the present invention is trans-Entacapone with a purity not less than 99.80% and containing an amount of cis-Entacapone or any other impurity not more than 0.10%, or trans-Entacapone of pharmaceutical grade.

In one embodiment, trans-Entacapone obtained according to the present invention is in a polymorph form A, as described in EP 0 426 468, the content of which, relative to the characterization of this polymorph as disclosed in Table I and Table II, is herein incorporated by reference.

The purity as well as the amounts of impurities are determined by a validated HPLC method, as depicted below in the experimental part.

### EXPERIMENTAL PART

### Example 1 - purification of trans-Entacapone (inventive method)

A flask is charged with 76.7 g of crude Entacapone (of purity 99,7% and containing 1,0% of cis-Entacapone) in 383.5 ml of toluene and 536.9 ml of acetone and the mixture is heated at reflux until complete dissolution.

7.7 g of Acticarbon are then added and the mixture is stirred at reflux for 30 minutes, is filtered while still hot and the filter cake is washed with 3x38.4 ml of acetone.

The filtrate is added to the Entacapone mixture, which is heated again until dissolution. Then, 375.83 ml of acetone are distilled off with Dean-Stark under atmospheric pressure, until an internal temperature of 75-80°C is reached.

Heating is switched off and the mixture is kept hot (hot oil bath) until the product starts to precipitate. After 1 hour under stirring the oil bath is removed and the mixture is allowed to cool to 20°C.

After 1 hour at 20°C under stirring, the mixture is colled to 0°C and is stirred for additional 3 hours, then it is filtered and the solid cake is washed with a cool mixture of 38.4 ml toluene and 19 ml acetone.

The product so obtained is dried in an oven at 45°C for 10-12 hours, to give trans-Entacapone in a yield of 84% and with a purity of 99.88%. Cis-Entacapone was not detected (lower than 0,02%) and major impurity 0,04%.

### Example 2 - Comparative example

25 g of crude Entacapone (of purity 96,8% amd containing 0.16% of cis-Entacapone) are suspended in 850 ml of toluene, the suspension is refluxed until dissolution and 2.5 g of Acticarbon are added. The mixture is then hot filtered and the cake is washed with toluene. About 600 ml of toluene are distilled off under atmospheric pressure, the mixture is allowed to cool to room temperature and subsequently to 0°C-10°C for 6 hours. The solid is filtered and washed with cool toluene, to give trans-Entacapone in a yield of 38% and with a purity of 99.4% and containing 0,10% of cis-Entacapone.

### Example 3 - Comparative example

88.5 g of crude Entacapone (of purity 97,2% and containing 1.32% of cis-Entacapone) are suspended in 1770 ml of ethyl acetate, then the mixture is refluxed and stirred until dissolution. 8.9 g of Acticarbon are added,the mixture is stirred and hot filtered and the cake if washed with hot ethyl acetate. The solvent is distilled off under atmospheric pressure until the product precipitates, then is mixture is cooled to room temperature and subsequently to 10°C for 6 hours. The solid is filtered and washed with 44 ml of ethyl acetate, to give trans-Entacapone in a yield of 85% and a purity of 99.1% and containing 0,25% of cis-Entacapone.

### Example 4 - Comparative example

25 g of crude Entacapone (of purity 97,4% and containing 1.0% of cis-Entacapone) are suspended in 125 ml of toluene and 125 ml of ethanol. The mixture is refluxed until complete dissolution, then 2.5 g of Acticarbon are added, it is filtered and the cake is washed with toluene/ethanol 1:1. The mixture is allowed to cool to room temperature, then it is cooled to 0°C-5°C and it is stirred for 24 hours. The solid is filtered and washed with 25 ml of a 1/1 mixture of toluene/ethanol. Trans-Entacapone is isolated in a yield of 88% and with a purity of 99.2% and 0,10% of cis-Entacapone.

### Validated HPLC method for determining the purity of Entacapone and impurity profile

### Chromatographic conditions:

| | | | |
|---|---|---|---|
| Column | YMC ODS-AQ, 250x4.6 mm, 5 µm or equivalent | | |
| Column temp. | 25°C | | |
| Mobile phase A | H₃PO₄ 0.1% in water | | |
| Mobile phase B | acetonitrile | | |

| Gradient | t(min) | %A | %B |
|---|---|---|---|
| | 0 | 80 | 20 |
| | 1 | 80 | 20 |
| | 20 | 20 | 80 |
| | 30 | 20 | 80 |
| Flux | 1 ml/min | | |
| Detector | UV at 220 nm | | |
| Injection | equal volumes (about 1 µl) | | |
| Diluent | water/acetonitrile 1:1 v/v | | |
| Analysis duration | 30 minutes | | |

### Peak table:

| **Compound/impurity** | **RT (min)** | **RRT** | **FC** |
|---|---|---|---|
| Amide | 6.9 | 0.48 | 2.8 |
| Aldheyde | 9.9 | 0.68 | 0.8 |
| Cis-Entacapone | 14.0 | 0.97 | 1 |
| Trans-Entacapone | 14.5 | 1 | 1 |
| Toluene | 20.3 | 1.40 | |
| Xilene | 21.8-23.2 | | |

### Procedure

Injecting the sample solution and recording the chromatogram.

Evaluating the percentage of trans-Entacapone and of the known and unknown impurities with the method of the area percent distribution. Recording the percentage of trans-Entacapone with two decimal figures.

Entacapone purity and impurities content are calculated as area percentage.

The method of purification of Entacapone according to the invention provides trans-Entacapone in a high yield, very high purity and in particular with a very reduced amount of cis-Entacapone and of all the other impurities (Entacapone of pharmaceutical grade). None of the prior art methods is able to furnish all the above advantages together.

In addition, the amount of solvent is reduced with respect to the crystallization methods known so far, that also implies an increased productivity of the method.

## Claims

1. A method for the purification of crude Entacapone, comprising crystallizing the said crude Entacapone from a mixture of solvents **characterized in that** it is composed of toluene and acetone, wherein the said mixture of toluene and acetone has a toluene/acetone ratio of between 5:1.5 and 5:6 and wherein the amount of toluene with respect to Entacapone is of between 4.8 and 5.2 volumes of toluene per gram of Entacapone.

2. The method of claim 1, which comprises:
a) dissolving by heating crude Entacapone in a mixture toluene/acetone;
b) distilling off an amount of acetone until an internal temperature of 75-80°C is reached in the Entacapone solution;
c) allowing the Entacapone solution to cool down slowly until Entacapone precipitates;
d) cooling down the Entacapone suspension and isolating pure Entacapone.

3. The method of claim 2, wherein the said dissolving by heating in step a) is conducted at reflux temperature.

4. The method of claim 2 or 3, wherein the said step a) further comprises adding an amount of active carbon in the heated Entacapone solution, keeping under stirring at reflux for a time span and filtering the active carbon off.

5. The method of claim 4, wherein the said amount of active carbon is a 5-15% and the said time span is of between 20-40 minutes.

6. The method of claim 4 or 5, wherein the said active carbon filter cake is washed with acetone and the resulting acetone phase is added to the Entacapone solution.

7. The method of any one of claims 2 to 6, wherein in step b), the amount of acetone distilled off is such as to obtain a final toluene/acetone ratio of between 5:1 and 5:3.

8. The method of any one of claims 2 to 7, wherein step d) further comprises:
- cooling down the Entacapone suspension to room temperature or to 20°C and keeping under stirring for 30-90 minutes, and subsequently
- cooling down the Entacapone suspension to between -5°C and 5°C and keeping under stirring for 2 to 4 hours.

## Patentansprüche

1. Verfahren zur Reinigung von unaufbereitetem Entacapon, umfassend Kristallisieren des unaufbereiteten Entacapons aus einer Mischung von Lösungsmitteln, dadurch charakterisiert, dass sie zusammengesetzt ist aus Toluol und Aceton, wobei die Mischung aus Toluol und Aceton ein Toluol/Aceton-Verhältnis von zwischen 5:1,5 und 5:6 aufweist, und wobei der Anteil des Toluols in Bezug auf Entacapon von zwischen 4,8 und 5,2 Volumina Toluol pro Gramm Entacapon ist.

2. Verfahren nach Anspruch 1, welches umfasst:
a) Lösen durch Erwärmen von unaufbereitetem Entacapon in einer Mischung Toluol/Aceton;
b) Abdestillieren eines Anteils von Aceton bis eine interne Temperatur von 75-80°C in der Entacapon-Lösung erreicht ist;
c) Zulassen, dass die Entacapon-Lösung langsam abkühlt bis Entacapon ausfällt;
d) Abkühlen der Entacapon-Suspension und Isolieren von reinen Entacapon.

3. Verfahren nach Anspruch 2, wobei das Lösen durch Erwärmen in Schritt a) bei Rückflusstemperatur durchgeführt wird.

4. Verfahren nach Anspruch 2 oder 3, wobei der Schritt a) weiterhin Zugeben eines Anteils aktiven Kohlenstoffs in die erwärmte Entacapon-Lösung, Halten unter Rühren bei Rückfluss für eine Zeitdauer und Abfiltrieren des aktiven Kohlenstoffs umfasst.

5. Verfahren nach Anspruch 4, wobei der Anteil aktiven Kohlenstoffs 5-15% ist und die Zeitdauer von zwischen 20-40 Minuten ist.

6. Verfahren nach Anspruch 4 oder 5, wobei der aktive Kohlenstofffilterkuchen mit Aceton gewaschen wirt und die resultierende Acetonphase zu der Entacapon-Lösung zugegeben wird.

7. Verfahren nach einem der Ansprüche 2 bis 6, wobei in Schritt b) der Anteil von abdestilliertem Aceton dergestalt ist, um ein finales Toluol/ Aceton-Verhältnis von zwischen 5:1 und 5:3 zu erhalten.

8. Verfahren nach einem der Ansprüche 2 bis 7, wobei Schritt d) weiterhin umfasst:
- Abkühlen der Entacapon-Suspension auf Raumtemperatur oder auf 20 °C und Halten unter Rühren für 30-90 Minuten und anschließend
- Abkühlen der Entacapon-Suspension auf zwischen -5°C und 5°C und Halten unter Rühren für 2 bis 4 Stunden.

## Revendications

1. Procédé pour la purification de l'Entacapone brute, comprenant la cristallisation de ladite Entacapone brute dans un mélange de solvants **caractérisé en ce qu'**il est composé de toluène et d'acétone, où ledit mélange de toluène et d'acétone a un rapport toluène/acétone entre 5:1,5 et 5:6 et où la quantité de toluène par rapport à l'Entacapone est entre 4,8 et 5,2 volumes de toluène par gramme d'Entacapone.

2. Procédé selon la revendication 1, qui comprend :
a) la dissolution par chauffage d'Entacapone brute dans un mélange toluène/acétone ;
b) le retrait par distillation d'une quantité d'acétone jusqu'à ce qu'une température interne de 75-80°C soit atteinte dans la solution d'Entacapone ;
c) la mise à refroidir de la solution d'Entacapone lentement jusqu'à ce que l'Entacapone précipite ;
d) le refroidissement de la suspension d'Entacapone et l'isolement d'Entacapone pure.

3. Procédé selon la revendication 2, où ladite dissolution par chauffage dans l'étape a) est conduite à la température de reflux.

4. Procédé selon la revendication 2 ou 3, où ladite étape a) comprend en outre l'addition d'une quantité de carbone activé dans la solution d'Entacapone chauffée, le maintien sous agitation à reflux pendant une durée et le retrait du carbone activé par filtration.

5. Procédé selon la revendication 4, où ladite quantité de carbone activé est 5-15 % et ladite durée est entre 20-40 minutes.

6. Procédé selon la revendication 4 ou 5, où ledit gâteau de filtration de carbone activé est lavé avec l'acétone et la phase d'acétone résultante est ajoutée à la solution d'Entacapone.

7. Procédé selon l'une quelconque des revendications 2 à 6, où dans l'étape b), la quantité d'acétone retirée par distillation est telle qu'elle permet d'obtenir un rapport toluène/acétone final entre 5:1 et 5:3.

8. Procédé selon l'une quelconque des revendications 2 à 7, où l'étape d) comprend en outre :
- le refroidissement de la suspension d'Entacapone à la température ambiante ou à 20°C et le maintien sous agitation pendant 30-90 minutes, puis
- le refroidissement de la suspension d'Entacapone entre -5°C et 5°C et le maintien sous agitation pendant 2 à 4 heures.
